(19) Europäisches Patentamt
European Patent Office
Office européen des brevets

(11) EP 0 635 502 B1

(12) EUROPÄISCHE PATENTSCHRIFT

(45) Veröffentlichungstag und Bekanntmachung des Hinweises auf die Patenterteilung:
**07.05.1997 Patentblatt 1997/19**

(51) Int Cl.[6]: **C07D 307/92**, C07C 69/738, A61K 7/46

(21) Anmeldenummer: **94110722.9**

(22) Anmeldetag: **11.07.1994**

# (54) Naphthofuranderivate als Riech- und Aromastoffe

Naphthofuran derivatives as odoriferous substances and flavouring substances

Dérivés de naphthofuranne comme matières odorantes et aromatisants

(84) Benannte Vertragsstaaten:
**CH DE ES FR GB IT LI NL**

(30) Priorität: **23.07.1993 CH 2234/93**

(43) Veröffentlichungstag der Anmeldung:
**25.01.1995 Patentblatt 1995/04**

(73) Patentinhaber: **GIVAUDAN-ROURE (INTERNATIONAL) S.A.**
**CH-1214 Vernier, Genève (CH)**

(72) Erfinder: **Naegeli, Peter**
**CH-5430 Wettingen (CH)**

(74) Vertreter: **Urech, Peter, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 170 955**
**WO-A-82/00030**
**CH-A- 680 444**

- **K. BAUER UND D. GARBE 'Common Fragrance and Flavor Materials' 1985 , VCH VERLAGSGESELLSCHAFT , WEINHEIM DE Seite 3, Absatz "Odor and structure"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 635 502 B1

**Beschreibung**

Die vorliegende Erfindung betrifft neue Naphthofuranderivate der allgemeinen Formel

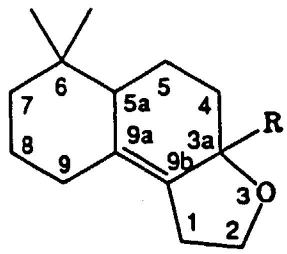

I

worin R Wasserstoff, Methyl oder Ethyl darstellt.

Die neuen Verbindungen der Formel I sind wertvolle Ambra-Riechstoffe und die Erfindung betrifft demgemäss auch die Verwendung dieser Riechstoffe in Parfum- und Aromakompositionen. Die Erfindung betrifft zudem die Herstellung der Verbindung der Formel I sowie auch der erwähnten Parfum- und Aromakompositionen.

Die obige Formel I umfasst sämtliche Stereoisomeren, d.h. u.a. die stereoisomeren Verbindungen der nachfolgenden Formeln Ia und Ib, sowie auch deren jeweilige Enantiomere:

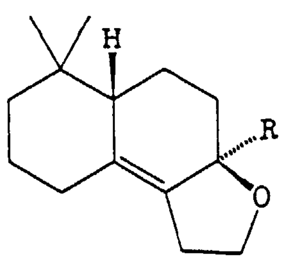

Ia

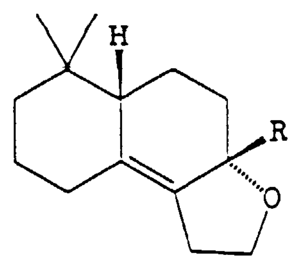

Ib

worin R die obige Bedeutung hat.

Die sensorischen Eigenschaften der Verbindungen der Formel I fallen in den Bereich der Ambrakörper.

Aus der Strukturklasse der hydrierten Naphtho[2,1-b]furane sind bereits eine Anzahl in der Literatur beschrieben, beispielsweise von G. Ohloff in "Fragrance Chemistry" Ed. E.T. Theimer, 1982, Academic Press, Seiten 535 ff. Auf dem Riechstoffmarkt ist aus dieser Strukturklasse bisher jedoch nur AMBROX® (Ohloff, loc. cit, Verbindung 21 und EP 170 955 (A3), beinhaltend ein Verfahren zur Herstellung der racemischen Verbindung; das hier ebenfalls erwähnte iso-AMBROX ist geruchlich verschieden von AMBROX), dank seiner Geruchsstärke, und auch teilweise wegen seiner Naturidentität, sehr beliebt und in grossen Mengen eingesetzt worden. Dieses Produkt charakterisiert den typischen warm-holzig-tabakartigen Teilaspekt der Grauen Ambra (Ambregris), dem sehr knapp vorhandenen und teuren Naturmaterial.

Es blieb daher seit jeher ein Bedürfnis, weitere, starke Ambrakörper zu finden und zu entwickeln; nach Möglichkeit solche, die auch die anderen Geruchsnuancen der Grauen Ambra aufweisen, ohne deren Haftfestigkeit zu ermangeln (zur Beschreibung des Geruchs der Grauen Ambra, siehe vorhergehend erwähnte Literaturstelle).

Derartige Verbindungen, d.h. Ambrakörper, werden nun erfindungsgemäss durch die Verbindungen der obigen Formel I zur Verfügung gestellt. Es wurde überraschend gefunden, dass diese Verbindungen einerseits sehr wohl die Geruchsstärke von z.B. AMBROX® erreichen, dazu aber auch noch geruchlich den Gesamtaspekt des Ambregris viel typischer wiedergeben.

Die erfindungsgemässen Verbindungen der Formel I können in einem grossen Bereich von Riechstoffkompositionen für die Parfümerie, die Kosmetik und auch für Aromen, insbesondere Tabakaromen, verwendet werden. Sie vertragen sich, d.h. sie harmonieren mit sämtlichen bekannten, in der Parfumerie- und Aromenkompositorik verwendeten Materialien wie beispielsweise:

- <u>Naturprodukte</u>, wie etwa Baummoos-Absolue, Basilikumöl, Agrumenöle (wie Bergamotteöl, Mandarinenöl, usw.),

Mastix-Absolue, Myrtenöl, Palmarosaöl, Patchouliöl, Petitgrainöl, Wermutöl, Lavendelöl, Rosenöl, Jasminöl, Ylang-Ylangoel oder Sandelholzöl usw.;

- Alkohole, wie Farnesol, Geraniol, Linalool, Nerol, Phenylethylalkohol, Rhodinol, Zimtalkohol, cis-3-Hexenol, Menthol, α-Tocopherol usw.;
- Aldehyde, wie Citral, α-Hexylzimtaldehyd, Hydroxycitronellal, LILIAL® (p-tert.Butyl-α-methyl-dihydrozimtaldehyd), Methylnonylacetaldehyd, Phenylacetaldehyd, Anisaldehyd, Vanillin usw.;
- Ketone, wie Allyljonon, α-Jonon, β-Jonon, Isoraldein (Isomethyl-α-jonon), Verbenon, Nootkaton, Geranylaceton usw.;
- Ester, wie Allyl-phenoxyacetat, Benzyl-salicylat, Cinnamylpropionat, Citronellyl-acetat, Decylacetat, Dimethylbenzylcarbinylacetat, Aethylacetoacetat, Aethyl-acetylacetat, cis-3-Hexenyl-isobutyrat, Linalylacetat, Methyl-dehydrojasmonat, Styrallylacetat, Vetiverylacetat, Benzylacetat, cis-3-Hexenyl-salicylat, Geranylacetat usw.;
- Lactone, wie γ-Undecalacton, δ-Decalacton, Pentadecan-15-olid usw.;
- Acetale, wie VIRIDIN® (1,1-Dimethoxy-2-phenyläthan) usw.;
- verschiedene, in der Parfumerie oft benutzte Komponenten, wie Indol, p-Menthan-8-thiol-3-on, Methyleugenol, Eugenol, Anethol usw.

Die Verbindungen der Formel I lassen sich in weiten Grenzen einsetzen, die z.B. von ca. 0,01 bis ca. 90 Gew.% reichen können. Für Riechstoffkompositionen liegen die Konzentrationen vorzugsweise zwischen etwa 0,1 und etwa 5 Gew.%. Als Komponenten für Aromenkompositionen können die Verbindungen der Formel I z.B. zur Verbesserung, Modifizierung und/oder Verstärkung von Fruchtaromen beitragen, oder auch als holzige und tabakartige Aromen für Nahrungs- und Genussmittel verwendet werden, wobei hier die Konzentration im Endprodukt vorzugsweise im Bereich von etwa 0,01 bis etwa 500 ppm (parts per million) zu liegen kommt.

Die Verbindungen I können, wie erwähnt, bei der Herstellung von Riechstoff- bzw. Aromenkompositionen unter Verwendung einer breiten Palette bekannter Riechstoffe bzw. Riechstoffgemische oder Aromastoffe oder deren Gemische verwendet werden. Bei der Herstellung solcher Kompositionen können die oben aufgeführten bekannten Riechstoffe bzw. Riechstoffgemische nach an sich bekannter Art und Weise verwendet werden.

Die erfindungsgemässen Verbindungen der Formel I können in an sich bekannter Weise hergestellt werden, beispielsweise gemäss dem nachfolgenden Reaktionsschema I. In den in diesem Schema aufgeführten Formeln hat der Substituent R die oben angegebene Bedeutung und der Substituent $R^1$ bedeutet niederes Alkyl. Dieser Ausdruck wiederum bedeutet geradkettige oder verzweigte Alkylgruppen mit 1 bis 6 Kohlenstoffatomen wie z.B. Methyl, Ethyl, Propyl, Isopropyl, Butyl, Isobutyl, tert.Butyl und dergleichen.

Die Cyclisierung des 1,4-Diols - unter Wasserabspaltung - zum Aether kann - in der dem Fachmann bekannten Weise - sauer oder, unter Derivatisierung des primären Alkohols, z.B. als Mesylat, auch basisch erfolgen.

Die als Ausgangsmaterial verwendeten Verbindungen der Formel IV, worin $R^1$ die vorhergehende Bedeutung hat, sind neue Verbindungen und ebenfalls Gegenstand der vorliegenden Erfindung. Sie können in an sich bekannter Weise hergestellt, beispielsweise wie in den Schemata II und III dargestellt. Die im Schema I aufgeführten Verbindungen der Formeln IIIa, IIIb und IIa sind ebenfalls neue Verbindungen.

**Schema I**

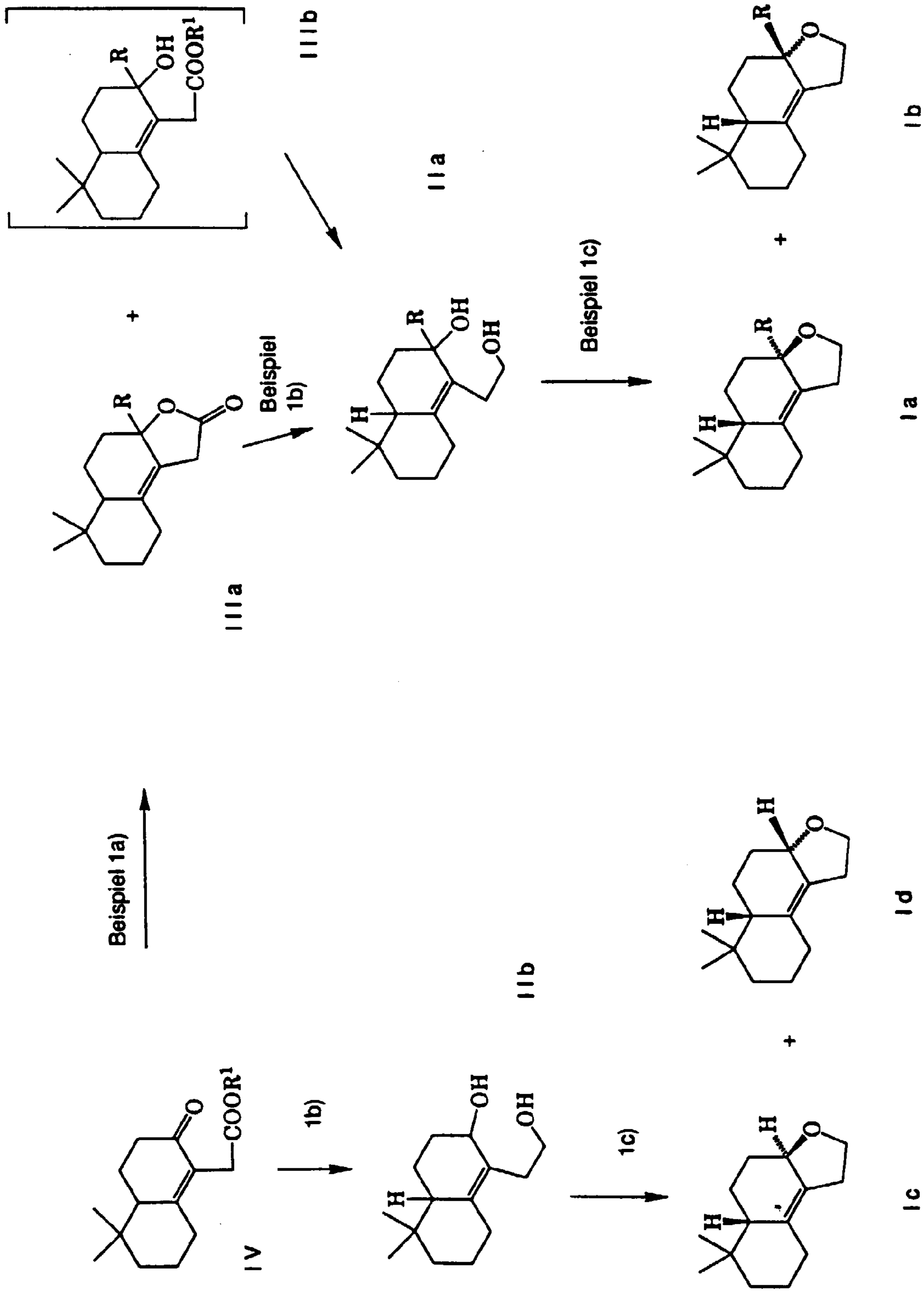
III b
II a
I b
+
Beispiel 1c)
Beispiel 1b)
III a
I a
Beispiel 1a)
II b
I d
IV
1b)
1c)
I c

## Schema II

VII VIII

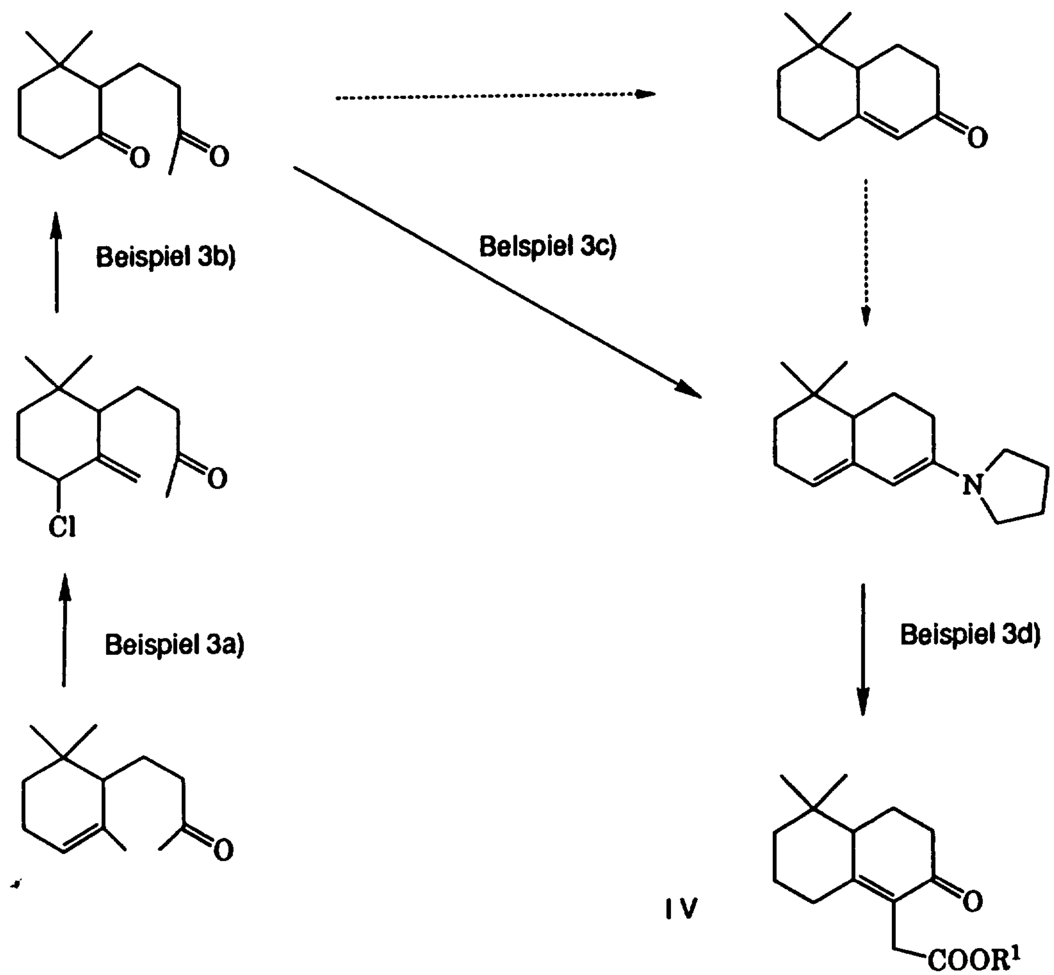


VI IX

V IV

**Schema III**

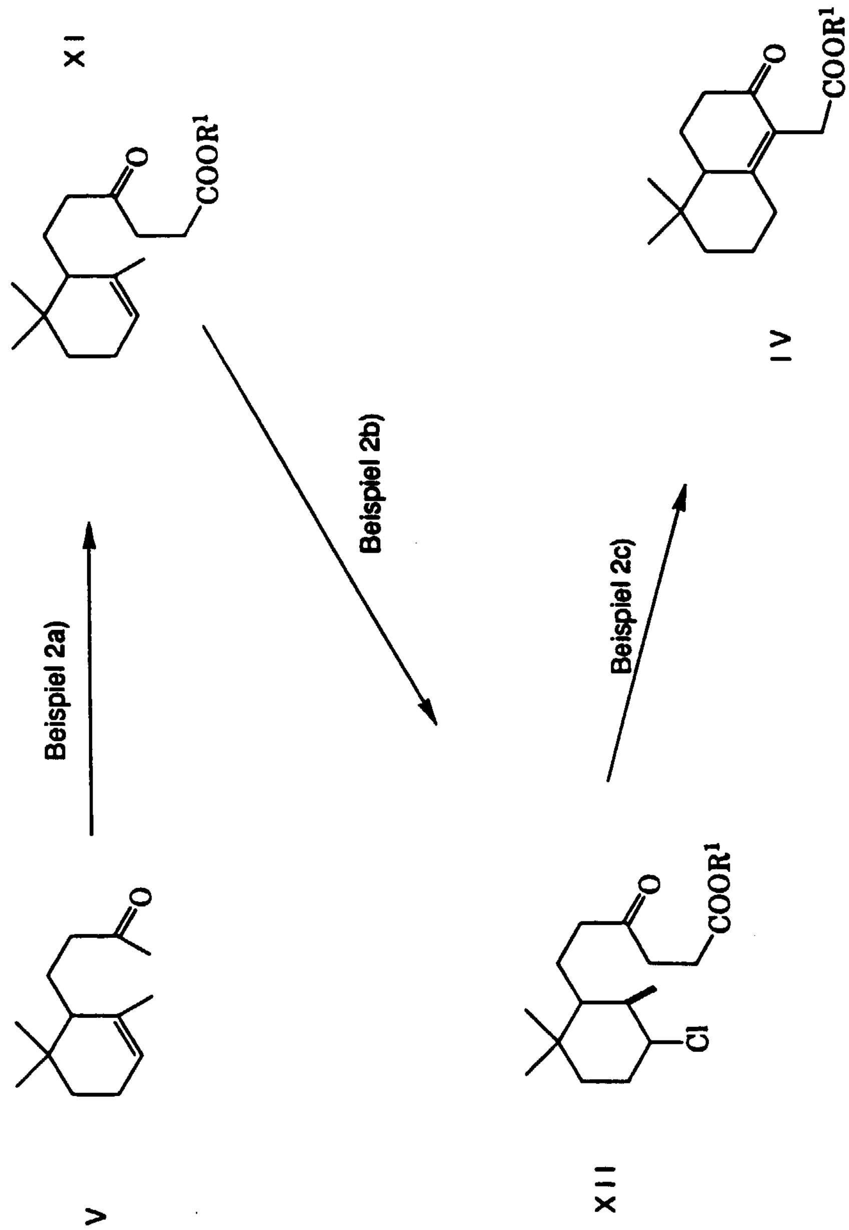


XI, IV, V, XII

Die nachfolgenden Beispiele dienen zur Illustrierung der Erfindung und stellen in keiner Weise irgendeine Beschränkung dar. Sämtliche Reaktionen werden unter Inertgas durchgeführt. Die Temperaturen sind in ° Celsius angegeben.

Beispiel 1

a) In einem 1,5 L Vierhalskolben, versehen mit Rührer, Thermometer, Kühler und Septumstopfen, werden unter Argon 30 g (103 mMol) tert.Butyl-2-[5,5-dimethyl-2-oxo-2,3,4,4a,5,6,7,8-octahydronaphth-1-yl]-acetat in 530 ml 1,2-Dimethoxyäthan vorgelegt. Die Lösung wird auf -30° abgekühlt und bei -30° bis -20° mit 154 ml (154 mMol) Methyllithium-Lösung (1N in Ether) innert 10 Minuten versetzt. Das erhaltene Gemisch wird bei -30° weitergerührt und nach 3 Stunden werden weitere 15,4 ml (15,4 mMol) Methyllithium-Lösung zugetropft und das Kühlbad entfernt. Nach 22 Stunden wird die Reaktionslösung auf ca. 1,5 L gesättigte $NH_4$-Cl-Lösung gegossen, wobei die vorher dunkelrote Lösung gelb wird. Hierauf werden die Phasen getrennt und die wässrige Phase dreimal mit Hexan extrahiert. Die vereinigten organischen Phasen werden mit Wasser und mit gesättigter NaCl-Lösung gewaschen, über $MgSO_4$ getrocknet und eingedampft. Man erhält 23,57 g Rohprodukt in Form eines dunkelgelben Öls, welches durch Chromatographie an Silicagel gereinigt wird. Man erhält so ein reines 1:1-Gemisch von (3aRS,5aSR)- und (3aSR,5aSR)-3a,6,6-trimethyl-1,2,3a,4,5,5a,6,7,8,9-decahydronaphtho[2,1-b]furan-2-on.

NMR (400 MHz) $CDCl_3$ + TMS f. Peak 1: u.a. 3.17 ppm (1H, aufgespaltenes d, J=20); 1.40 ppm (9H,s); 1.006 ppm (3H,s); 0.793 ppm (3H,s)

NMR (400 MHz) $CDCl_3$ + TMS f. Peak 2: u.a. 3.11 ppm (1H,d,J=20); 2.035 ppm (1H,dt,$J_1$=11,$J_2$=4); 1.415 ppm (9H,gespaltenes s); 0.95 ppm (3H,s); 0.747 ppm (3H,s).

b) In einem 1,5 L Vierhalskolben, versehen mit Rührer, Thermometer, Rückflusskühler und Tropftrichter, werden unter Argon 3,8 g (98 mMol) $LiAlH_4$ in 100 ml Diethylether vorgelegt. Dann werden bei Raumtemperatur 23 g (98 mMol) des gemäss a) hergestellten Gemisches in 500 ml Diethylether innert 25 Minuten zugetropft. Hierauf wird die graue Suspension während 2 Stunden unter Rückfluss erhitzt, dann in einem Eisbad abgekühlt und mit 200 ml gesättigter $K_2CO_3$-Lösung hydrolysiert. Nach Trennung der Phasen, wird die wässerige Phase dreimal mit ether extrahiert. Die vereinten organischen Phasen werden mit gesättigter NaCl-Lösung und Wasser neutral gewaschen, über $MgSO_4$ getrocknet und eingedampft. Man erhält 21,23 g Diastereomerengemisch von 1-(2-Hydroxyethyl)-2,5,5-trimethyl-2,3,4,4a,5,6,7,8-octahydronaphthalen-2-ol als gelbes, hochviskoses Öl.

IR (Film): 3320 (str.), 1460(m), 1390 + 1370(w), 1140(m), 1070 + 1040(m) $cm^{-1}$

IR ($CHCl_3$): 3600(m), 3380(str.), 1450(str.), 1380 + 1360(m), 1260(str.), 1060 + 1040(str.) $cm^{-1}$.

Nach chromatographischer Auftrennung an Silicagel (Ether:Hexan = 4:1) erhält man die zwei Diastereomeren in reiner, kristalliner Form:

Apolares Epimer (Smp. 82-88°C) ($CH_3$ u. H cis)

NMR ($CDCl_3$ + TMS, 200 MHz): 3.80 ppm (1H,ddd,$J_1$=10,$J_2$=5,$J_3$=4,5); 3.55 ppm (1H,td,$J_1$=10,$J_2$=4,5); 1.315 ppm (3H,s); 0.98 ppm (3H,s); 0.80 ppm (3H,s)

Polares Epimer (Smp. 116-130°C) ($CH_3$ u. H trans)

NMR ($CDCl_3$ + TMS, 200 MHz): 3.75 ppm (1H,ddd,$J_1$=10,$J_2$=6,$J_3$=4); 3.60 ppm (1H,td,$J_1$=10,$J_2$=5); 1.26 ppm (3H, s); 0.92 ppm (3H,s); 0.73 ppm (3H,s)

c) In einem 1,5 L Vierhalskolben, versehen mit Rührer, Thermometer, Kühler und Tropftrichter, werden unter Stickstoff 21 g (88 mMol) des gemäss b) hergestellten Diastereomerengemisches in 600 ml Methylenchlorid vorgelegt und in einem Eisbad abgekühlt. Dann werden innert 25 Minuten bei 5-7° 7,2 ml (88 mMol) Methansulfonsäurechlorid in 300 ml Pyridin zugetropft und das Gemisch bei 5° weiter gerührt. Nach 2,5 Stunden wird die orangefarbene Lösung in 750 ml Wasser gegossen und die Phasen werden getrennt. Die wässerige Phase wird zweimal mit Ether extrahiert. Die vereinten organischen Phasen werden zweimal mit Wasser, mit kalter 6N $H_2SO_4$ bis pH = 1, zweimal mit Wasser, einmal mit gesättigter $NaHCO_3$-Lösung und zweimal mit gesättigter NaCl-Lösung gewaschen, über $MgSO_4$ getrocknet, filtriert und eingedampft. Man erhält 21,05 g Rohprodukt in Form eines dunkelorangen Öls. Das Rohprodukt wird in 350 ml 2N KOH in Methanol gelöst und unter Rückfluss erhitzt. Nach 2 Stunden wird die Lösung mit 200 ml Wasser verdünnt und fünfmal mit je 100 ml Hexan extrahiert. Die vereinigten organischen Phasen werden mit gesättigter NaCl-Lösung neutralgewaschen, über $MgSO_4$ getrocknet, filtriert und eingedampft. Man erhält 17,44 g eines 1:1-Gemisches von (3aRS,5aSR)- und (3aSR,5aSR)-3a,6,6-trimethyl-1,2,3a,4,5,5a,6,7,8,9-decahydro-naphtho[2,1-b]-furan als dunkelgelbes Öl mit dem Geruch der Grauen Ambra (ambregris).

Die beiden reinen Isomeren (cis- und trans-Verbindung) können, wie vorhergehend beschrieben, aus den zwei reinen Diastereomeren gemäss b), hergestellt werden.

Cis-Verbindung: 3aβ,6,6-Trimethyl-1,2,3a,4,5,5aβ,6,7,8,9-decahydronaphtho[2,1-b]-furan.

$^{13}C$-NMR ($CDCl_3$): 3 x q bei 30.744, 22.624 und 21.290 ppm; 7 x t bei 64.189, 42.379, 33.618, 31.496, 27.625, 22.432, 20.700 ppm; d bei 44.016 ppm; 4 x s bei 134.744, 127.878, 78.488 und 36.477 ppm.

$^1H$-NMR: 400 MHz ($CDCl_3$ + TMS): 3.99 ppm (1H,ddd,$J_1$=9,$J_2$=9,$J_3$=6); 3.89 ppm (1H,ddd,$J_1$=9,$J_2$=9,$J_3$=6); 2.65 ppm (1H,d von t,$J_1$=15,$J_2$=7); 2.55 ppm (1H,dddd,$J_1$=13,$J_2$=13,$J_3$=6,$J_4$=3); 2.39 ppm (1H,breites d,J=14); 1.86 ppm (1H,d, ps.t,$J_1$=14,$J_2$=3); 1.13 ppm (3H,split s); 0.986 ppm (3H,s); 0.785 ppm (3H,s).

trans-Verbindung: 3aα,6,6-Trimethyl-1,2,3a,4,5,5aβ,6,7,8,9-decahydronaphtho[2,1-b]-furan.

MS: m/e = 220(1), 205(100), 190(1), 177(3), 163(4), 149(6), 137(5), 121(4), 105(5), 91(7), 79(5), 69(13), 55(6), 43(15).

IR (Film): 1450 + 1435(m), 1380(w), 1360(m), 1210(w), 1135(m), 1030(str) $cm^{-1}$.

$^{13}C$-NMR ($CDCl_3$): 3 x q bei 29.20, 22.996, 19.905 ppm; d bei 47.532 ppm; 7 x t bei 63.881, 42.120, 35.141, 30.745, 27.669, 22.382, 20.916 ppm; 4 x s bei 134.275, 128.449, 78.187, 34.875 ppm.

$^1H$-NMR: 400 MHz ($CDCl_3$ + TMS): 3.97 ppm (1H,ddd,$J_1$=8.5,$J_2$=8.5,$J_3$=6); 3.835 ppm (1H,ddd,$J_1$=8.5,$J_2$=8.5,$J_3$=6); 2.35 ppm (1H,dq,$J_1$=13,$J_2$=2); je 3H (s) bei 1.15, 0.93, 0.733 ppm.

In zum Vorhergehenden analoger Weise können, durch Reduktion von tert.Butyl-2-[5,5-dimethyl-2-oxo-2,3,4,4a,5,6,7,8-octahydro-naphth-1-yl]-acetat und Cyclisation der erhaltenen Diole, auch folgende Verbindungen hergestellt werden:

Cis-Verbindung: 6,6-Dimethyl-1,2,3aβ,4,5,5aβ,6,7,8,9-decahydronaphtho[2,1-b]-furan.

trans-Verbindung: 6,6-Dimethyl-1,2,3aα,4,5,5aβ,6,7,8,9-decahydronaphtho[2,1-b]-furan.

Beispiel 2

Das gemäss Beispiel 1 als Ausgangsmaterial verwendete tert.Butyl-2-[5,5-dimethyl-2-oxo-2,3,4,4a,5,6,7,8-octahydronaphth-1-yl]-acetat kann wie folgt hergestellt werden:

a) In einem 1,5 L Vierhalskolben, versehen mit Rührer, Thermofühler, Kühler und Septumstopfen, werden unter Argon 48 ml (0,36 Mol) Diisopropylamin in 800 ml Tetrahydrofuran vorgelegt und die Lösung mit einem Isopropanol/Trockeneis-Kühlbad abgekühlt. Bei -70° werden dann 222 ml (0,36 Mol) Butyllithium-Lösung (1,6M in Hexan) zugegeben und danach das Kühlbad für ca. 0,5 Stunden entfernt. Anschliessend wird erneut abgekühlt und bei -70° werden 75 ml (0,43 Mol) Hexamethylphosphortriamid zugegeben. Nach 20 Minuten werden bei -75° bis -72° 58,35g (0,30 Mol) Dihydro-α-Jonon, in etwas Tetrahydrofuran gelöst, zugetropft. Nach 1 Stunde werden dann noch 175,5 g (0,90 Mol) Bromessigsäure-tert.butylester innert 3 Minuten bei -75° bis -50° zugetropft. Nach etwa 70 Minuten bei -70° wird das Reaktionsgemisch in eine gesättigte $NH_4Cl$-Lösung (~ 1,5 L) gegossen, die Phasen werden getrennt und die wässerige Phase 4 mal mit Hexan extrahiert. Die vereinigten organischen Phasen werden mit gesättigter NaCl-Lösung neutralgewaschen, über $MgSO_4$ getrocknet, abfiltriert und am Rotationsverdampfer eingedampft. Man erhält 201,47 g Rohprodukt als hellgelbe Flüssigkeit. Nach zweimaliger Destillation, mit Hilfe eines Kurzweg-Filmverdampfers, zwecks Entfernung von Bromessigsäure-tert.Butyl-ester, erhält man 87,34 g 6-(2,6,6-Trimethylcyclohex-2-en-1-yl)-4-oxo-hexansäure-tert.butylester als gelbes Öl.
NMR ($CDCl_3$ + TMS) (400 MHz): 5.33 ppm (1H,m); 2.66 ppm (2H,dd,J=6) und 2.49 ppm (2H,dd,J=6); 2.47 ppm (1H,dd,$J_1$=8,$J_2$=6); 1.965 ppm (2H,m); 1.67 ppm (3H,split s); 1.44 ppm (9H,s); 1.13 ppm (1H,dt,$J_1$=13,$J_2$=5); 0.92 und 0.87 ppm (je breit 3H,s).
b) In einem 6 L Vierhalskolben, versehen mit Rührer, Thermometer, Kühler und Tropftrichter, werden 172 g (0,56 Mol) des gemäss a) hergestellten Ketoesters in 1,6 L Methylenchlorid vorgelegt und mit 330 g (2,3 Mol) Kaliumdihydrogenphosphat, gelöst in 1,6 L entionisiertem Wasser, versetzt. Die entstandene Mischung wird in einem Eisbad abgekühlt und dann werden innert 35 Minuten bei 10°-11° 406 ml (0,58 Mol) Natriumhypochlorit-Lösung (~11%ig) zugetropft. Anschliessend wird das Gemisch 3 Stunden im Eisbad weiter gerührt. Dann werden die Phasen getrennt und die wässerige Phase wird mit $CH_2Cl_2$ extrahiert. Die vereinigten organischen Phasen werden mit Wasser, gesättigter $NaHCO_3$-Lösung und nochmals mit Wasser gewaschen, über $MgSO_4$ getrocknet, filtriert und am Rotationsverdampfer bei höchstens 30° Badtemperatur eingedampft. Man erhält 193,13 g 6-(2-Methyliden-6,6-dimethyl-3-chlorocyclohex-1-yl)-4-oxo-hexansäure-tert.butylester als gelbes Öl. NMR: 200 MHz ($CDCl_3$ + TMS): 5.34 ppm und 4.78 ppm (je 1H, breite s); 4.475 ppm (1H,dd,$J_1$=7,$J_2$=5); 1.44 ppm (9H,s); 0.94 und 0.855 ppm (je 3H,s).
c) In einem Begasungsgefäss werden 190 g (0,55 Mol) 6-(2-Methyliden-6,6-dimethyl-3-chlorocyclohex-1-yl)-4-oxo-hexansäure-tert.butylester in 1,7 L Methanol gelöst, auf -70° abgekühlt und während 1 Stunde und 45 Minuten ozonisiert ($O_2$-Fluss = 200 L/h). In einem 4 L Vierhalskolben, versehen mit Rührer, Thermometer, Kühler und Tropftrichter werden 108 g (1,65 Mol) mit $CuSO_4$ aktiviertes Zink-Pulver, 200 ml entionisiertes Wasser und 150 ml einer Lösung von 115 g $K_2HPO_4$ in 300 ml $H_2O$ vorgelegt. Die kalte Reaktionsmischung der Ozonisierung wird rasch zugetropft und die entstandene, graue Suspension unter Rückfluss erhitzt. Nach 17 Stunden wird der Rest der $K_2HPO_4$-Lösung zugegeben und das Gemisch wird unter Rückfluss erhitzt. Nach 4 Tagen wird der pH-Wert des Gemisches mit gesättigter $K_2HPO_4$-Lösung auf ~7 eingestellt, nach 5 Tagen auf pH 7,5 und nach 6 Tagen auf pH 8. Nach total 11 Tagen wird die graue Mischung abkühlen gelassen und die Phasen werden getrennt. Die wässerige Phase wird 3 mal mit Hexan extrahiert. Die vereinigten organischen Phasen werden 3 mal mit gesättigter NaCl-Lösung neutralgewaschen, über $MgSO_4$ getrocknet und eingedampft. Man erhält 84,17 g tert. Butyl-2-[5,5-dimethyl-2-oxo-2,3,4,4a,5,6,7,8-octahydronaphth-1-yl]acetat als gelbes Öl.

Nach 2-maliger Destillation dieses Produktes bei 130° bzw. 170° und 0,267 mbar und Chromatographie an $SiO_2$ erhält man ein reines Produkt.
NMR: 200 MHz ($CDCl_3$ + TMS): 3.36 ppm (1H,s); 1.48 ppm (1H,dd,$J_1$=13,$J_2$=3); 1.42 ppm (9H,s); 1.055 ppm und 0.85 ppm (je 3H,s).

Das vorhergehend mit $CuSO_4$ aktivierte Zink-Pulver kann wie folgt hergestellt werden:

130 g Zink-Pulver werden mit 200 ml 0,01N HCl-Lösung aufgeschlämmt, abdekantiert und mit Wasser neutralgewaschen. Dann wird das Zink zu einer Lösung von 7,7 g $CuSO_4$ in 200 ml Wasser gegeben und während 2,5 Stunden gerührt. Danach wird das ZnCu abgenutscht, mit Wasser, Aceton und Ether nachgewaschen und im Trockenschrank bei Raumtemperatur getrocknet.

Beispiel 3

Das gemäss Beispiel 1 als Ausgangsmaterial verwendete tert.Butyl-2-[5,5-dimethyl-2-oxo-2,3,4,4a,5,6,7,8-octahydronaphth-1-yl]-acetat kann auch noch wie folgt hergestellt werden:

a) In einem 10 L Vierhalskolben, versehen mit Rührer, Thermometer, Kühler und Tropftrichter, werden 388 g (2 Mol) Dihydro-α-jonon in 2,2 L Methylenchlorid vorgelegt. Dann werden 660 g (4,6 Mol) Kaliumdihydrogenphosphat, in 3 L Wasser gelöst, zugegeben und die entstehende Emulsion im Eisbad abgekühlt. Bei ca. 10° werden innert 1,5 Stunden 1,6 L (2,3 Mol) Natriumhypochlorit-Lösung (~12%ig) zugegeben und nach ca. 3 Stunden bei 15° weitere 350 ml (0,5 Mol). Der pH-Wert der Mischung wird mit Essigsäure auf ~ 5,5 eingestellt und es wird weiter bei Raumtemperatur gerührt. Nach total 27 Stunden Reaktionszeit werden die Phasen getrennt und die wässerige Phase 4 mal mit Methylenchlorid extrahiert. Die vereinigten organischen Phasen werden 1 mal mit Wasser, 2 mal mit gesättigter $NaHCO_3$-Lösung und nochmals 3 mal mit Wasser neutralgewaschen, über $MgSO_4$ getrocknet, filtriert und eingedampft (Badtemperatur = Raumtemperatur). Man erhält 407,45 g 4-(3-Chloro-2-methyliden-6,6-dimethyl-cyclohex-1-yl)-butan-2-on als gelbe Flüssigkeit.
NMR: 200 MHz ($CDCl_3$ + TMS): 5.34 ppm (1H,s); 4.78 ppm (1H,s); 4.48 ppm (1H,dd,$J_1$=6.5,$J_2$=5); 2.12 ppm (3H, s); 0.943 und 0.86 ppm (s, je 3H).
b) In einem 3 L Begasungsgefäss werden 200 g (0,87 Mol) 4-(3-Chloro-2-methyliden-6,6-dimethyl-cyclohex-1-yl)-butan-2-on [hergestellt gemäss a)] in 1,7 L Methanol gelöst, auf ~ -70° abgekühlt und während 2 Stunden und 15 Minuten ozonisiert ($O_2$-Fluss = 300 L/h). Dabei wird die gelbe Lösung farblos und wird hierauf während 30 Minuten mit $O_2$ durchblasen.
In einem 4,5 L Vierhalskolben, versehen mit Rührer, Thermometer, Kühler und Tropftrichter, werden 165,5 g Zink-Pulver und 550 ml Wasser vorgelegt. Dann wird innert 5 Minuten die kalte Reaktionsmischung der Ozonisierung zugegeben und das Gemisch mit 25%iger $H_2SO_4$ auf pH 2,5 eingestellt, wobei die Temperatur auf 65° ansteigt. Die Mischung wird unter Rückfluss erhitzt, und der pH-Wert laufend mit $H_2SO_4$ bei 2-3 gehalten. Nach 3 Stunden wird die Mischung abgekühlt, das Zink-Pulver abgenutscht und die Lösung 4 mal mit Ether extrahiert. Die vereinten Etherphasen werden 1 mal mit gesättigter NaCl-Lösung, 2 mal mit gesättigter $NaHCO_3$-Lösung und nochmals 2 mal mit gesättigter NaCl-Lösung neutralgewaschen, über $MgSO_4$ getrocknet, filtriert und eingedampft. Man erhält 145 g 4-(3-Oxo-6,6-dimethylcyclohex-1-yl)-butan-2-on als gelbe Flüssigkeit.
NMR: 200 MHz ($CDCl_3$ + TMS): 2.58 ppm (1H,ddd,$J_1$=17,$J_2$=9,$J_3$=5.5); 2.11 ppm (3H,s); 1.085 ppm (3H,s); 0.785 ppm (3H,s).
c) In einem 250 ml Zweihalskolben, versehen mit Magnetrührer, Thermometer und Wasserabscheider mit Kühler, werden unter Stickstoff 8,5 g (43,3 mMol) 4-(3-Oxo-6,6-dimethylcyclohex-1-yl)-butan-2-on in 170 ml Cyclohexan vorgelegt. 3,57 ml (43,3 mMol) Pyrrolidin und 100 mg (0,53 mMol) p-Toluolsulfonsäure werden dann zugegeben und das Ganze unter Rückfluss erhitzt. Nach ~23 Stunden werden 0,73 g (5,3 mMol) Kaliumcarbonat zugesetzt und das Cyclohexan wird abdestilliert. Der Rückstand wird im Kugelrohr bei 100°-110° und 0,267 mbar destilliert und man erhält 10,34 g 5,5-Dimethyl-2-N-pyrrolidinyl-3,4,4a,5,6,7-hexahydronaphthalin.
H-NMR: 200 MHz ($CDCl_3$ + TMS): 5.125 ppm (1H,m); 4.90 ppm (1H,s); 0.985 ppm (3H,s); 0.79 ppm (3H,s).
d) In einem 250 ml Vierhalskolben, versehen mit Rührer, Thermometer, Kühler und Tropftrichter, werden unter Argon 9,3 g (40,2 mMol) 5,5-Dimethyl-2-N-pyrrolidinyl-3,4,4a,5,6,7-hexahydronaphthalin [erhalten gemäss c)] in 110 ml Propionitril vorgelegt. Dann werden 8,5 ml (48 mMol) N-Aethyldiisopropylamin und 6,6 ml (44,2 mMol) Bromessigsäure-tert.-butylester zugegeben und die dunkelgelbe Lösung unter Rückfluss erhitzt. Nach 10 Tagen wird die dunkelbraune Mischung abkühlen gelassen, im Eisbad mit 100 ml Essigsäure/Acetat-Puffer (pH 4,1) versetzt und 20 Minuten reagieren gelassen. Die Mischung wird dann 2 mal mit 100 ml Hexan extrahiert. Die organische Phase wird 10 mal mit 100 ml Wasser gewaschen, über $MgSO_4$ getrocknet, filtriert und eingedampft. Man erhält 9,93 g tert.Butyl-2-[5,5-dimethyl-2-oxo-2,3,4,4a,5,6,7,8-octahydronaphth-1-yl]-acetat.

Beispiel 4

In den Beispielen a) bis d) bezieht sich die jeweils an erster Stelle und in Klammern gesetzte Komponente (A) auf das Reaktionsprodukt von Beispiel 1c) d.h. auf das 1:1 Gemisch von (3aRS,5aSR)- und (3aSR,5aSR)-3a,6,6-Trimethyl-1,2,3a,4,5,5a,6,7,8,9-decahydro-naphtho[2,1-b]-furan (Verbindungen der Formeln 1a und 1b).

| a) Tabakaroma (Typ Virginia): | | | |
|---|---|---|---|
| | Gewichsteile: | | |
| (A) | .... | 30,0 | 30,0 |
| Thibetolid (Ω-Pentadecalacton) | 0,4 | 0,4 | 0,4 |
| Sandelholzöl | 10,0 | 10,0 | 10,0 |
| Zedernholzöl Virginia | 1,5 | 1,5 | 1,5 |

(fortgesetzt)

| a) Tabakaroma (Typ Virginia): | | | |
|---|---|---|---|
| | Gewichsteile: | | |
| α-Jonon | 0,1 | 0,1 | 0,1 |
| β-Jonon | 0,5 | 0,5 | 0,5 |
| Ketoisophoron | 2,0 | 2,0 | 2,0 |
| Essigsäure-geranylester | 0,5 | 0,5 | 0,5 |
| Oleoresin ex Virginia-Tabak | -- | -- | 100,0 |
| Ethylalkohol | 155,0 | 155,0 | 155,0 |
| Propylenglykol | 830,0 | 800,0 | 700,0 |
| | 1000,0 | 1000,0 | 1000,0 |

Durch das Zufügen von (A) in obigen Kompositionen wird die deutlich vorhandene süssliche, holzige Note reduziert.

Beim Verrauchen von aromatisiertem Tabak tritt im Rauch eine typische, tabakartige, teeartige/holzige Note hervor, die stark an Virginia Tabak erinnert.

| b) Herrenparfumbase, Typ Masculin: | |
|---|---|
| | Gewichtsteile: |
| (A) | 70,0 |
| Benzylacetat | 30,0 |
| Geranylacetat | 50,0 |
| Allyl-amyl-glycolat | 3,0 |
| Methyl-anthranylat | 1,0 |
| Basilikumöl | 10,0 |
| Bergamotte Essenz (Calabr.) | 200,0 |
| α-Methyljonon | 50,0 |
| Citronen Essenz (Argentin.) | 100,0 |
| Cumarin, rein krist. | 20,0 |
| Dihydromyrcenol | 80,0 |
| Estragonöl | 5,0 |
| EVERNYL® (Methyl-β-orcincarboxylat) | 3,0 |
| FIXOLIDE® (1,1,2,4,4,7-Hexamethyl-6-acetyl-1,2,3,4-tetrahydronaphthalin) | 60,0 |
| Gewürznelkenöl | 15,0 |
| HEDIONE® (Methyldihydrojasmonat) | 50,0 |
| Isoeugenol | 3,0 |
| Lavandinöl | 100,0 |
| Methyl-cedryl-keton | 60,0 |
| Muskatnussessenz | 20,0 |
| Patchouliessenz (eisenfrei) | 40,0 |
| Pêche (γ-Undecalacton) | 1,0 |
| Petitgrainöl Paraguay | 7,0 |
| Sandalore [3-Methyl-5-(2,3,3-dimethylcyclopent-3-en-1-yl)-pentan-2-ol] | 20,0 |
| Vanillin | 2,0 |
| | 1000,0 |

Durch die Anwesenheit von (A) in obiger Base, wird in einem entsprechenden semi-orientalischen Parfum (7%ige alkoholische Lösung mit Alkohol 90°), durch dessen trockenen und natürlichen Ambregris-Charakter, die holzige Note verstärkt und dem ganzen Akkord mehr Wärme in animalisch-tabakartiger Richtung verliehen.

| c) Parfumbase, für Seife geeignet, Typ blumig: | |
|---|---|
| | Gewichtsteile: |
| (A) | 10,0 |
| Benzylacetat | 40,0 |
| Geranylacetat | 40,0 |
| 3-cis-Hexenylacetat | 2,0 |
| p-tert.Butyl-cyclohexylacetat | 60,0 |
| Zimtalkohol | 40,0 |
| Phenylethylalkohol | 100,0 |
| α-Hexylzimtaldehyd | 150,0 |
| Phenylacetaldehyd 85%/APE | 1,0 |
| Aubépine (Anisaldehyd) | 5,0 |
| Bergamotte-Essenz (Calabr.) | 60,0 |
| Zedernholzessenz (Virginia) | 50,0 |
| Dimethylbenzyl-carbinyl-butyrat | 5,0 |
| Citronellol extra | 40,0 |
| Cumarin krist. | 15,0 |
| δ-Decalacton | 4,0 |
| Dipropylenglycol | 20,0 |
| Aethylvanillin 10% DIP | 2,0 |
| Eugenol (rein) | 15,0 |
| EVERNYL® | 3,0 |
| FIXOLIDE® | 50,0 |
| Galbanumessenz | 1,0 |
| Hydroxycitronellal | 30,0 |
| Indolen | 2,0 |
| LILIAL® | 40,0 |
| Mandarinessenz | 10,0 |
| Methyl-Diantilis (Aethylvanillyl-methyläther) | 10,0 |
| Nonadyl | 30,0 |
| Amylsalicylat | 50,0 |
| Benzylsalicylat | 80,0 |
| α-Terpinol | 30,0 |
| Undecavertol (4-Methyl-dec-3-en-5-ol) | 5,0 |
| | 1000,0 |

In obiger Komposition führt die Anwesenheit von (A) zu einer warmen, animalisch-ambrigen Tonalität des Typs natürliche Graue Ambra, unterstützt die würzige Note und verstärkt den Tabakcharakter.

| d) Parfumbase für Kosmetika: | |
|---|---|
| | Gewichtsteile: |
| (A) | 50,0 |
| Benzylacetat | 40,0 |
| Dimethylbenzyl-carbinylacetat | 40,0 |
| Geranylacetat | 30,0 |
| Phenylethylalkohol | 100,0 |
| Bergamotte-Essenz (Calabr.) | 100,0 |
| α-Methyljonon | 80,0 |
| Cyclohexal | 40,0 |
| Dipropylenglycol | 50,0 |

(fortgesetzt)

| d) Parfumbase für Kosmetika: | |
|---|---|
| | Gewichtsteile: |
| Eugenol | 20,0 |
| Galaxolide 50 DEP (1,3,4,6,7,8-Hexahydro-4,6,6,7,8,8-hexamethyl-cyclopenta-γ-2-benzopyran) | 40,0 |
| Gardenol (Methyl-phenyl-carbinylacetat) | 10,0 |
| Geraniol (rein) | 50,0 |
| HEDIONE® (Dihydrojasmonat) | 40,0 |
| Heliotropin krist. | 20,0 |
| Hydroxycitronellal | 50,0 |
| Methyl-cedrylketon | 60,0 |
| Methylisoeugenol | 10,0 |
| Moschusketon | 50,0 |
| Benzylsalicylat | 100,0 |
| Thibetolid | 20,0 |
| | 1000,0 |

In diesem blumigen Akkord für Crèmes verleiht die Anwesenheit von (A) dem Ganzen eine sinnlichere Hautpflegenote durch die Verbindung ihres Ambregris-Charakters mit den verwendeten Moschuskörpern.

## Patentansprüche

**1.** Naphthofuranderivate der allgemeinen Formel

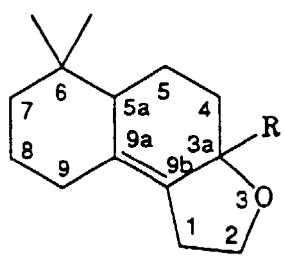

I

worin R Wasserstoff, Methyl oder Ethyl darstellt.

**2.** Naphthofüranderivate gemäss Anspruch 1 der allgemeinen Formeln

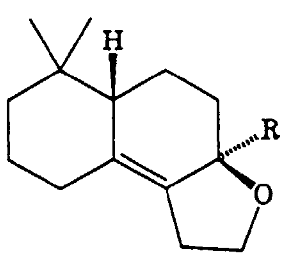

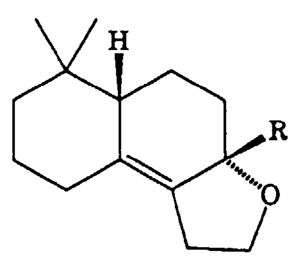

Ia und Ib

worin R Wasserstoff, Methyl oder Ethyl darstellt,
sowie deren jeweilige Enantiomere.

3. Naphthofuranderivate der Formel Ia und Ib gemäss Anspruch 2, worin R Methyl bedeutet.

4. Verbindungen der allgemeinen Formel

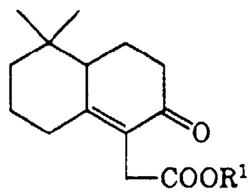

IV

worin $R^1$ niederes Alkyl darstellt.

5. Verfahren zur Herstellung der Verbindungen der Formel I gemäss Anspruch 1, 2 oder 3, dadurch gekennzeichnet, dass man eine Verbindung der Formeln

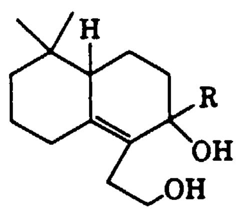

IIa oder

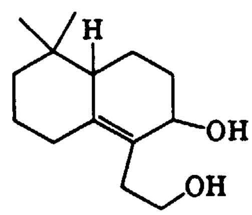

IIb

cyclisiert.

6. Riech- und/oder Geschmackstoffkomposition, gekennzeichnet durch einen Gehalt an einer Verbindung gemäss Anspruch 1, 2 oder 3.

7. Verwendung der Naphthofuranderivate gemäss einem der Ansprüche 1 bis 3 in Parfüm- und Aromakompositionen.

## Claims

1. Naphthofuran derivatives of the general formula

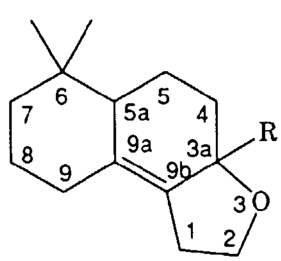

I

wherein R represents hydrogen, methyl or ethyl.

2. Naphthofuran derivatives according to claim 1 of the general formulae

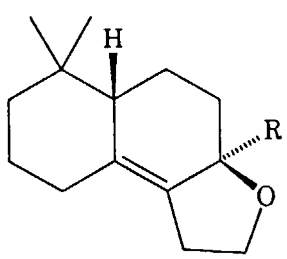

Ia and Ib

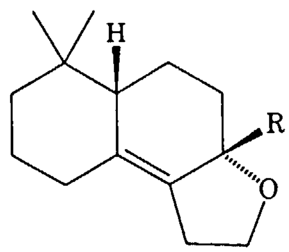

wherein R represents hydrogen, methyl or ethyl, as well as their respective enantiomers.

**3.** Naphthofuran derivatives of formulae 1a and 1b according to claim 2, wherein R signifies methyl.

**4.** Compounds of the general formula

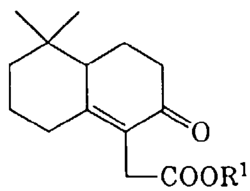

IV

wherein $R^1$ represents lower alkyl.

**5.** A process for the manufacture of the compounds of formula I according to claim 1, 2 or 3, characterized by cyclizing a compound of the formula

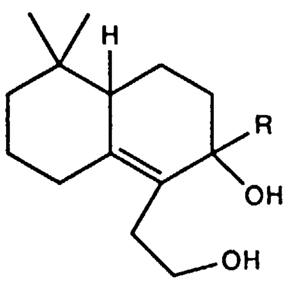

IIa or

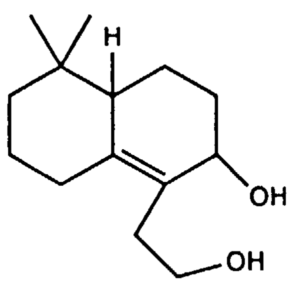

IIb

**6.** An odorant and/or flavour composition, characterized by a content of a compound according to claim 1, 2 or 3.

**7.** The use of the naphthofuran derivatives according to any one of claims 1 to 3 in perfume and flavour compositions.

**Revendications**

**1.** Dérivés de naphtofuranne de formule générale

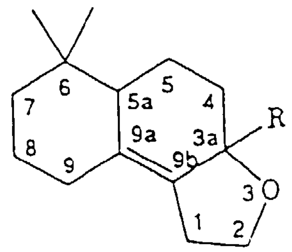

I

dans laquelle R représente un hydrogène, un méthyle ou un éthyle.

**2.** Dérivés de naphtofuranne selon la revendication 1 de formules générales

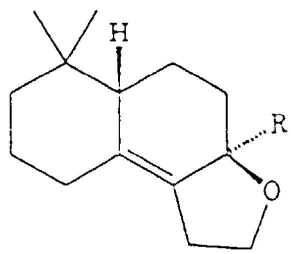

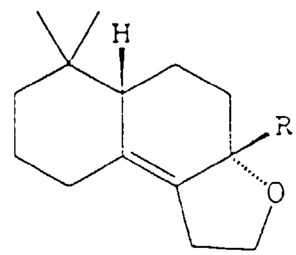

Ia et Ib

dans lesquelles R est un hydrogène, un méthyle ou un éthyle, ainsi que leurs énantiomères respectifs.

**3.** Dérivés de naphtofuranne de formule 1a et 1b selon la revendication 2, dans laquelle R représente un méthyle.

**4.** Composés de formule générale

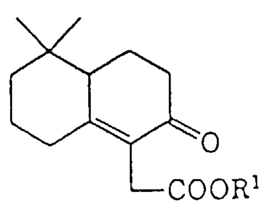

IV

dans laquelle $R^1$ représente un alkyle inférieur.

**5.** Procédé de préparation des composés de formule I selon la revendication 1, 2 ou 3, caractérisé en ce qu'on cyclise un composé de formules

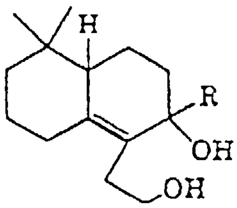 IIa ou 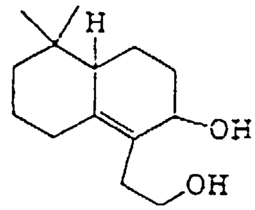 IIb

6. Compositions de parfum et/ou gustatives caractérisées en ce qu'elles contiennent un composé selon la revendication 1, 2 ou 3.

7. Application des dérivés de naphtofuranne selon l'une des revendications 1 à 3 dans des compositions de parfums et d'arômes.